# EUROPÄISCHE PATENTANMELDUNG

(11) **EP 1 783 488 A1**
(43) Veröffentlichungstag der Anmeldung: **09.05.2007**
(21) Anmeldenummer: 06022762.6
(22) Anmeldetag: 01.11.2006
(51) Int. Cl.: G01N 33/02, B07C 5/00

(54) **Vorrichtung zur Bestimmung der Qualität einer Getreideprobe**

(30) Priorität: 02.11.2005 DE 102005052769
(71) Anmelder: Bitzer Wiegetechnik GmbH, 31135 Hildesheim (DE)
(72) Erfinder: Bitzer, Siegmar, 31188 Holle (DE)
(74) Vertreter: Lins, Edgar

(57) **Zusammenfassung**

Zur Bestimmung der Qualität einer Getreideprobe, enthält eine Vorrichtung
- eine Leseeinrichtung zum Einlesen eines der Probe zugeordneten Identifizierungscodes (22)
- eine Aufgabeeinrichtung (1) für die Probe
- eine mit der Aufgabeeinrichtung (1) verbundene erste Wägeeinrichtung (5) für die aufgegebene Probe
- eine nach Abschluss einer Wägung öffnende Verschlussanordnung (6) am unteren Ende der ersten Wägeeinrichtung (5)
- eine unter der ersten Wägeeinrichtung (5) angeordnete Sichteranordnung (7) zum Abtrennen und pneumatischen Abführen von Leichtstoffen
- eine unter der Sichteranordnung (7) angeordnete Abtrennanordnung (9) für gegenüber Getreidekörnern größere Abmessungen aufweisende Fremdkörper
- eine unter der Abtrennanordnung (9) angeordnete Siebanordnung (10) zum Trennen von Kleinkom und Ganzkorn
- eine zweite Wägeeinrichtung (15) zur Wägung des Kleinkorn- und des Ganzkornanteils
- eine Abführvorrichtung (16) zum Entsorgen des Kleinkomanteils
- eine Übergabevorrichtung (14) zur Übergabe des Ganzkornanteils an eine Transportvorrichtung (17) zum Transportieren des Ganzkornanteils zu einer Aufnahme (18) eines Ganzkornanalysegeräts (19)
- eine Abführvorrichtung (23) zum Abführen des Ganzkornanteils nach Beendigung von mit dem Ganzkornanalysegerät (19) vorgenommenen Analysen
- einen zentralen Rechner, dem die Messwerte der Wägeeinrichtungen (5, 15) und des Ganzkomanalysegeräts (19) zuführbar sind und
- eine Ausgabeeinrichtung (20) für vom zentralen Rechner gelieferte Qualitätswerte der Probe.

## Beschreibung

Die Erfindung betrifft eine Vorrichtung zur Bestimmung der Qualität einer Getreideprobe.

Derartige Vorrichtungen werden insbesondere in Labors von Annahmestellen für geerntetes Getreide eingesetzt.

Das einer Annahmestelle auf Straßenfahrzeugen - aber auch auf Schienenfahrzeugen - angelieferte Getreide muss einer Qualitätsprüfung unterzogen werden, um einerseits den Wert - und damit den Preis - und andererseits die Lagerfähigkeit des angelieferten Getreides zu bestimmen. Ein Handhabungsproblem besteht darin, dass das mit dem Getreide beladene Fahrzeug über eine entsprechende Fahrzeugwaage gefahren wird, um das Gesamtgewicht festzustellen. Aus einem Vergleich mit dem Leergewicht des Fahrzeugs nach dem Abladen des Getreides lässt sich dann die angelieferte Brutto-Getreidemenge bestimmen. Während dieses Wägevorganges wird wenigstens eine Probe des angelieferten Getreides entnommen und im Labor untersucht. Die hierfür relevanten Daten ergeben sich aus der Feststellung des Anteils des Besatzes des Getreides, also der Feststellung des Anteils von Fremdstoffen, der Feststellung des Kleinkornanteils und somit der Feststellung des für die Weiterverarbeitung zu hochqualitativem Mehl brauchbaren Ganzkornanteils

Für diese Vorgänge werden Probenreiniger verwendet, die zur Abtrennung von Leichtstoffen mit einem Luftsichter und mit Siebanordnungen zur Abtrennung von Fremdstoffen mit größeren Abmessungen als Ganzkomabmessungen und zur Trennung von Ganzkorn und Kleinkorn ausgerüstet sind.

Die in dem Probenreiniger eingegebene Probe wird im Labor verwogen. Nach der Probenreinigung werden die gereinigte Ganzkornmenge einerseits und die gereinigte Kleinkornmenge andererseits ebenfalls verwogen. Die Differenz zwischen der Gesamt-Probenmenge und der Gesamtmenge von Ganzkorn und Kleinkorn erlaubt die Ermittlung des Besatzes des Korns, während mit den Gewichten des Kleinkornanteils und des Ganzkornanteils das Verhältnis von Ganzkorn zu Kleinkorn bestimmbar ist.

Anschließend wird beispielsweise das separierte Ganzkorn in ein Kornanalysegerät eingefüllt und dort mit bekannten Messmethoden, insbesondere Nah-Infrarot-Transmission (NIT) und Nah-Infrarot-Reflektion (NIR), untersucht. Dabei lassen sich beispielsweise die Parameter Feuchte, Proteingehalt, Sedimentationswert, Klebergehalt, Fettanteil usw. je nach Bedarf ermitteln.

Da ein Abladen des angelieferten Getreides erst erfolgen kann, wenn die Qualität des Getreides - und damit seine Verwendung und sein in Frage kommender Lagerort - und ggf. eine erforderliche Nachbehandlung (Trocknung) feststeht, stellt die im Labor durchzuführende Untersuchung einen zeitlichen Flaschenhals für die Abwicklung der Anlieferung des Getreides dar. Die Probenanalyse erfordert deutlich mehr Zeit als der Wägevorgang mit der Fahrzeugwaage, der in größenordnungsmäßig einer Minute abgeschlossen sein kann. Aufgrund der Labortätigkeit ergibt sich somit eine nicht unerhebliche Wartezeit für das mit Getreide beladene Fahrzeug, bevor es entladen werden kann.

Der vorliegenden Erfindung liegt die Aufgabe zugrunde, die Bearbeitungszeit im Labor für die Untersuchung der Getreideprobe zu verkürzen, um so die Wartezeit für die beladenen Fahrzeuge zu verringern.
Zur Lösung dieser Aufgabe enthält eine Vorrichtung zur Bestimmung der Qualität einer Getreideprobe
- eine Leseeinrichtung zum Einlesen eines der Probe zugeordneten ldentifizierungscodes
- eine Aufgabeeinrichtung für die Probe
- eine mit der Aufgabeeinrichtung verbundene erste Wägeeinrichtung für die aufgegebene Probe
- eine nach Abschluss einer Wägung öffnende Verschlussanordnung am unteren Ende der ersten Wägeeinrichtung
- eine unter der ersten Wägeeinrichtung angeordnete Sichteranordnung zum Abtrennen und pneumatischen Abführen von Leichtstoffen
- eine unter der Sichteranordnung angeordnete Abtrennanordnung für gegenüber Getreidekörnern größere Abmessungen aufweisende Fremdkörper
- eine unter der Abtrennanordnung angeordnete Siebanordnung zum Trennen von Kleinkorn und Ganzkorn
- eine zweite Wägeeinrichtung zur Wägung des Kleinkorn- und des Ganzkornariteils
- eine Abführvorrichtung zum Entsorgen des Kleinkornanteils
- eine Übergabevorrichtung zur Übergabe des selektierten Kornanteils an eine Transportvorrichtung zum Transportieren des Kornanteils zu einer Aufnahme eines Kornanalysegeräts
- eine Abführvorrichtung zum Abführen des selektierten Kornanteils nach Beendigung von mit dem Kornanalysegerät vorgenommenen Analysen
- einen zentralen Rechner, dem die Messwerte der Wägeeinrichtungen und des Kornanalysegeräts zuführbar sind und
- eine Ausgabeeinrichtung für vom zentralen Rechner gelieferte Qualitätswerte der Probe.

Die erfindungsgemäße Vorrichtung ermöglicht erstmalig einen vollautomatischen Ablauf der für die Bestimmung der Qualität einer Getreideprobe erforderlichen Messungen bis zur Ausgabe der gemessenen bzw. berechneten Qualitätswerte, die beispielsweise durch ein ausgedrucktes Etikett erfolgen kann. Der Ausdruck der Werte auf einem selbstklebenden Etikett ist besonders zweckmäßig, wenn in üblicher Weise von der untersuchten Probenmenge eine Rückstellprobe abgezweigt worden ist, die dann mit den ermittelten Analysedaten - vorzugsweise mit einem selbstklebenden Etikett - etikettiert werden kann.

Für die automatische Durchführung aller erforderlichen Bestimmungen können in der erfindungsgemäßen Vorrichtung ein herkömmlicher Probenreiniger und ein herkömmliches Ganzkornanalysegerät verwendet werden. Diese sind in der erfindungsgemäßen Vorrichtung in geschickter Weise mit der ersten und der zweiten Wägeeinrichtung sowie einer - vorzugsweise pneumatischen - Transportvorrichtung kombiniert. Ein zentraler Rechner übemimmt dabei die Ablaufsteuerung in der Vorrichtung und ist mit einer Leseeinrichtung zur Zuordnung eines Identifikationscodes zu der untersuchten Probe kombiniert. Die mit dem zentralen Rechner vorgenommene Ablaufsteuerung kann dabei so eingerichtet sein, dass die Wägung einer neuen, identifizierten Probe und die anschließende Probenreinigung bereits beginnen kann, wenn die vorhergehende Probe die zweite Wägeeinrichtung verlassen hat, also dem Kornanalysegerät zugeführt ist. Auf diese Weise ist es möglich, mit der erfindungsgemäßen Vorrichtung eine Untersuchungs-Taktzeit von etwa einer Minute einzuhalten. Die etwa eine Minute benötigt das Komanalysegerät, um die für die Probenanalyse erwünschten Messwerte zu erstellen. Somit ermöglicht die erfindungsgemäße Vorrichtung eine Durchführung der Probenanalyse mit einem Analysentakt, der dem Wägetakt der Fahrzeugwaage in etwa entspricht, sodass aufgrund der Laboruntersuchungen für die Getreideprobe entstehende Wartezeiten weitgehend vermieden werden.

Die erfindungsgemäße Vorrichtung enthält ferner vorzugsweise eine Starteinrichtung, die nach dem Einlesen des Identifizierungscodes ein Startsignal zur Aktivierung der ersten Wägeeinrichtung generiert. Dadurch ist sichergestellt, dass die Identifizierung der Probe stattgefunden hat, bevor die Verarbeitung der Probe zur Durchführung der Messungen und Analysen beginnt. Femer kann dabei sichergestellt werden, dass zunächst eine ausreichende Rückstellmenge in den Probenbehälter, vorzugsweise einen Probenbeutel, abgefüllt worden ist.

Die Reinigung des Getreidekoms und die Klassifizierung in Kleinkorn und Ganzkorn erfolgt in herkömmlicher Weise, wobei mit dem Probenreiniger die zweite Wägeeinrichtung kombiniert ist. Diese weist vorzugsweise getrennte Aufnahmekammern für das Kleinkorn und das vom Kleinkorn separierte Ganzkorn auf, die mit separat steuerbaren Entleerungsvorrichtungen versehen sind. Grundsätzlich wäre es möglich, die zweite Wägeeinrichtung in Form von zwei Waagen für die beiden Aufnahmekammern auszubilden. In weniger aufwändiger und in einfacherer Weise kommt die zweite Wägeeinrichtung jedoch mit nur einer Waage aus, indem mit dieser Waage zwei Wägungen durchgeführt werden, nämlich eine gemeinsame Wägung des Ganzkom- und Kleinkornanteils in den beiden Aufnahmekammern und eine zweite Wägung nach der Entleerung der Aufnahmekammer für das Kleinkorn zur Ermittlung des Gewichts des gereinigten und separierten Ganzkoms. Selbstverständlich ist es auch denkbar, die Aufnahmekammer für das Ganzkorn zu entleeren und das Gewicht des Kleinkornanteils zu bestimmen, bevor dieser Anteil entsorgt wird. Die Entleerungsvorrichtungen können dabei Entleerungsböden sein, die beispielsweise als Schieber oder Klappen ausgebildet sind.

Es ist möglich, die erfindungsgemäße Vorrichtung durch weitere Trenn-, Klassifizierungs- und Wägeeinrichtungen zu ergänzen, um besondere Korngrößen und -zusammensetzungen zu dem Kornanalysegerät zu transportieren.

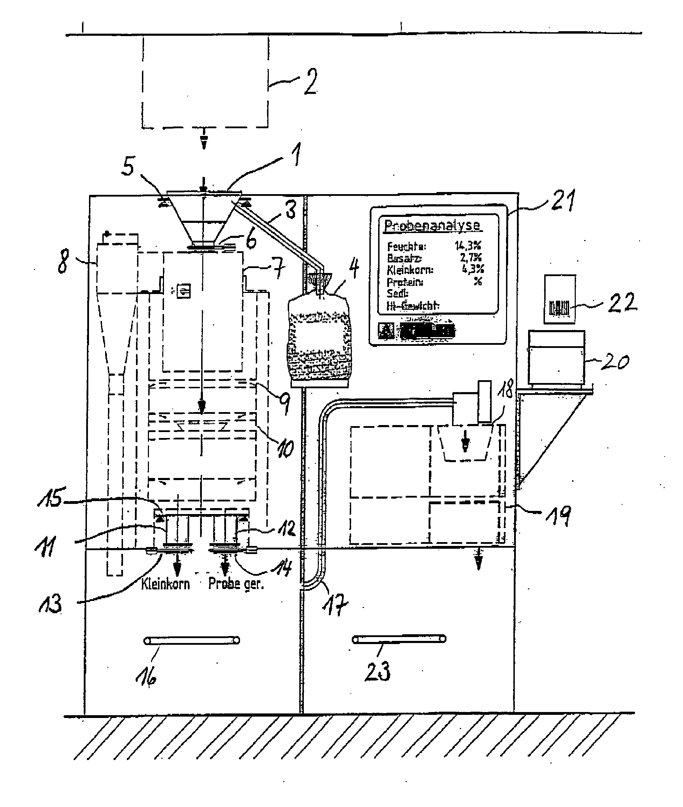

Die Erfindung soll im Folgenden anhand eines in der Zeichnung schematisch dargestellten Ausführungsbeispiels näher erläutert werden.

Die einzige Zeichnung lässt eine Aufgabeeinrichtung 1 in Form eines Aufgabetrichters erkennen, mit der Getreidekom einer Probe einfüllbar ist. Hierfür kann der Vorrichtung ein Probenteiler 2 zur automatischen Abzweigung einer geeigneten Probenmenge vorgeschaltet sein. Die Aufgabeeinrichtung 1 ist mit einem eine Füllvorrichtung bildenden Füllrohr 3 verbunden, über das eine Rückstellprobe des zu untersuchenden Korns in einen als Probenbeutel ausgebildeten Probenbehälter 4 abgezweigt wird.

Nachdem der Probenbehälter 4 für die Rückstellprobe gefüllt ist, erfolgt eine Wägung der eingefüllten Probe mittels einer ersten Wägeeinrichtung 5, in die der Aufgabetrichter der Aufgabeeinrichtung 1 eingehängt ist An seiner Unterseite weist der Aufgabetrichter eine steuerbare Verschlussanordnung 6 auf, die von einem die Ablaufsteuerung bewirkenden (nicht dargestellten) zentralen Rechner geöffnet wird, wenn der Wiegevorgang der ersten Wägeeinrichtung 5 abgeschlossen ist. Dadurch fällt das Getreidekom aus der Aufgabeeinrichtung 1 in eine Sichteranordnung 7, in der ein Luftstrom 7 als Gegenstrom zu der Fallrichtung des Getreidekoms ausgebildet ist. Der Luftstrom wird in einen Nebenbehälter 8 geleitet, in dem die vom Luftstrom mitgerissenen und von dem Getreidekom getrennten Leichtstoffe, wie Staub und Grannen, mittels eines (nicht dargestellten) Zyklons gesammelt und nach unten abgeleitet werden.

Unterhalb der Sichteranordnung 7 fällt das Getreidekorn auf eine Abtrennanordnung 9, in der sich Durchgangsöffnungen für die Dimension der Getreidekörner befinden, sodass Frontkörper, die eine darüber hinausgehende Dimension aufweisen, auf der Siebanordnung verbleiben und seitlich abgeführt werden können.

Das durch die Abtrennanordnung hindurchtretende Ganz- und Kleinkorn fällt auf eine darunter angeordnete Siebanordnung 10, in der durch geeignete, konisch verlaufende Transportspalte eine räumliche Trennung von dem großen Ganzkorn und dem deutlich kleineren Kleinkorn bewirkt wird. Dadurch wird erreicht, dass das Kleinkorn in eine Aufnahmekammer 11 und das Ganzkorn in eine seitlich davon getrennte Aufnahmekammer 12 gelangt. Die Aufnahmekammern 11, 12 sind jeweils an ihrem Boden mit je einer steuerbaren Entleerungsvorrichtung 13, 14 versehen, sodass die Aufnahmekammern 11, 12 separat voneinander entleerbar sind. Die Aufnahmekammern 11, 12 bilden einen Teil einer zweiten Wägeeinrichtung 15, mit der erfindungsgemäß der Gewichtsanteil des Kleinkorns und des Ganzkoms festgestellt werden. Die zweite Wägeeinrichtung 15 führt - vom zentralen Rechner gesteuert - zwei Wägungen aus, nämlich eine Wägung, in der beide Aufnahmekammern 11, 12 mit dem Kleinkorn bzw. dem Ganzkorn gefüllt sind, sodass sich ein Gesamtgewicht des Getreidekorns ermitteln lässt. Aus der Differenz zu dem Bruttogewicht der Probe, das mit der ersten Wägeeinrichtung 5 festgestellt worden ist, lässt sich der Besatzanteil des im Reinigungsvorgang entfernten Besatzes feststellen.

Anschließend wird die Entleerungsvorrichtung für die Aufnahmekammer 11 für das Kleinkorn geöffnet, sodass das Kleinkorn über eine Abtransportvorrichtung 16 entsorgt werden kann. Eine erneute Wägung mit der zweiten Wägeeinrichtung 15 ermöglicht nun die Feststellung des Gewichtes des Ganzkoms und (durch Differenzbildung) auch des Gewichtes des bereits entsorgten Kleinkorns.

Über die Entleerungsvorrichtung 14 der Aufnahmekammer 12 für das Ganzkorn wird das Ganzkorn nunmehr mittels einer pneumatischen Transportvorrichtung 17 in eine Aufnahme 18 eines als Ganzkornanalysegerät 19 ausgebildeten Kornanalysegeräts übergeben. Nach der Befüllung des Ganzkornanalysegeräts 19 wird dieses gestartet, sodass nunmehr die mit dem NIR- und/oder NIT-Verfahren ermittelbaren Parameter festgestellt werden können.

Die Ergebnisse werden dem zentralen Rechner zugeleitet, der seinerseits einen an der Vorrichtung vorhandenen Drucker 20 steuert, dem mit einer (nicht dargestellten) Etikettenzuführeinrichtung ein Selbstklebe-Etikett zuführbar ist, das mit den ermittelten Qualitätswerten bedruckt wird. In der Zeichnung ist ein bedrucktes Etikett 21 schematisch dargestellt, wie es auf den Probenbehälter 4 aufgeklebt wird, um die Rückstellwerte mit den bei der Eingangsüberprüfung festgestellten relevanten Qualitätswerten zu kennzeichnen.

Die Zeichnung lässt erkennen, dass der Drucker 20 zugleich als Lesegerät für einen der Probe zugeordneten Identifizierungscode 22 ausgebildet sein kann, der hier in Form eines Barcodes dargestellt ist. Auch der Identifizierungscode, der auf einem Selbstklebe-Etikett ausgedruckt sein kann, wird zweckmäßigerweise auf den gefüllten Probenbehälter 4 aufgeklebt, sodass eine unmittelbare Beziehung der in dem Probenbehälter 4 enthaltenen Rückstellprobe zu einer elektronisch abgelegten Dokumentation der durchgeführten Messung hergestellt werden kann.

Nach der Durchführung der Analyse in dem Ganzkomanalysengerät 19 wird der Ganzkornanteil mit einer Abtransportvorrichtung 23 automatisch entsorgt

Die Ablaufsteuerung wird vorzugsweise so vorgenommen, dass eine neue Wägung einer neuen Probe mit der ersten Wägeeinrichtung 5 starten kann, wenn die vorhergehende Probe aus den Aufnahmebehältem 11, 12 der zweiten Wägeeinrichtung 15 entnommen worden ist, also der Ganzkornanteil mittels der Transportvorrichtung 17 zum Ganzkomanalysengerät 19 transportiert wird bzw. worden ist.

Auf diese Weise gelingt eine deutlich verschachtelte Durchführung von Probenanalysen mit wenigstens zwei Proben zur gleichen Zeit.

## Patentansprüche

1. Vorrichtung zur Bestimmung der Qualität einer Getreideprobe, enthaltend
- eine Leseeinrichtung zum Einlesen eines der Probe zugeordneten Identifizierungscodes (22)
- eine Aufgabeeinrichtung (1) für die Probe
- eine mit der Aufgabeeinrichtung (1) verbundene erste Wägeeinrichtung (5) für die aufgegebene Probe
- eine nach Abschluss einer Wägung öffnende Verschlussanordnung (6) am unteren Ende der ersten Wägeeinrichtung (5)
- eine unter der ersten Wägeeinrichtung (5) angeordnete Sichteranordnung (7) zum Abtrennen und pneumatischen Abführen von Leichtstoffen
- eine unter der Sichteranordnung (7) angeordnete Abtrennanordnung (9) für gegenüber Getreidekörnern größere Abmessungen aufweisende Fremdkörper
- eine unter der Abtrennanordnung (9) angeordnete Siebanordnung (10) zum Trennen von Kleinkorn und Ganzkorn
- eine zweite Wägeeinrichtung (15) zur Wägung des Kleinkom- und des Ganzkornanteils
- eine Abführvorrichtung (16) zum Entsorgen des Kleinkornanteils
- eine Übergabevorrichtung (14) zur Übergabe eines selektierten Kornanteils an eine Transportvorrichtung (17) zum Transportieren des Kornanteils zu einer Aufnahme (18) eines Kornanalysegeräts (19)
- eine Abführvorrichtung (23) zum Abführen des selektierten Kornanteils nach Beendigung von mit dem Kornanalysegerät (19) vorgenommenen Analysen
- einen zentralen Rechner, dem die Messwerte der Wägeeinrichtungen (5, 15) und des Kornanalysegeräts (19) zuführbar sind und
- eine Ausgabeeinrichtung (20) für vom zentralen Rechner gelieferte Qualitätswerte der Probe.

2. Vorrichtung nach Anspruch 1, ferner enthaltend eine Füllvorrichtung (3) für eine Rückstellmenge in einen Probenbehälter (4) vor einer Aktivierung der ersten Wägeeinrichtung (5).

3. Vorrichtung nach Anspruch 1 oder 2, ferner enthaltend eine Starteinrichtung, die nach dem Einlesen des Identifizierungscodes (22) ein Startsignal zur Aktivierung der ersten Wägeeinrichtung (5) generiert.

4. Vorrichtung nach einem der Ansprüche 1 bis 3, bei der die zweite Wägeeinrichtung (15) getrennte Aufnahmekammern (11, 12) für das Kleinkorn und das vom Kleinkorn separierte Ganzkorn aufweist, die mit separat steuerbaren Entleerungsvorrichtungen (13, 14) versehen sind.

5. Vorrichtung nach Anspruch 4, bei der die Entleerungsvorrichtungen (13, 14) Entleerungsböden sind.

6. Vorrichtung nach einem der Ansprüche 1 bis 5, bei der die Transportvorrichtung (17) eine pneumatische Transportvorrichtung ist.

7. Vorrichtung nach einem der Ansprüche 1 bis 6, bei der der zentrale Rechner zugleich als Steuereinrichtung für die Abläufe in der Vorrichtung eingerichtet ist.

8. Vorrichtung nach Anspruch 7, bei der der zentrale Rechner zur Steuerung der ersten Wägeeinrichtung (5) für eine neue Probe eingerichtet ist, wenn die vorhergehende Probe die zweite Wägeeinrichtung (15) verlassen hat.

9. Vorrichtung nach einem der Ansprüche 1 bis 8, bei der die Ausgabeeinrichtung (20) einen Drucker zum Ausdrucken der Qualitätswerte der Probe aufweist.

10. Vorrichtung nach Anspruch 9, bei der mit dem Drucker (20) eine Etikettenzuführeinrichtung verbunden ist.
